# EUROPÄISCHE PATENTANMELDUNG

(11) **EP 4 497 792 A1**
(43) Veröffentlichungstag der Anmeldung: **29.01.2025**
(21) Anmeldenummer: 24190457.2
(22) Anmeldetag: 23.07.2024
(51) Int. Cl.: C09D 13/00, B43K 19/18, A61K 8/02, A61K 8/19, A61K 8/26, A61K 8/36, A61K 8/37, A61Q 1/02, B43K 19/00, B43K 19/02

(54) **MINE ODER KREIDE FÜR SCHREIB-, MAL- UND/ODER KOSMETIKZWECKE, STIFT MIT EINER MINE UND VERFAHREN ZUR HERSTELLUNG EINER MINE ODER KREIDE**

(30) Priorität: 24.07.2023 EP 23187376
(71) Anmelder: Faber- Castell AG, 90546 Stein (DE)
(72) Erfinder: Heuler, Baukis, 90471 Nürnberg (DE); Pöllmann, Christian, 90765 Fürth (DE); Lugert, Gerhard, 90431 Nürnberg (DE)
(74) Vertreter: Meissner Bolte Nürnberg

(57) **Zusammenfassung**

Die Erfindung betrifft eine biologisch abbaubare und/oder kompostierbare Mine oder Kreide für Schreib-, Mal- und/oder Kosmetikzwecke enthaltend wenigstens einen Fettsäureester, wenigstens eine Metallseife und/oder Seife und wenigstens ein Farbmittel, wobei der wenigstens eine Fettsäureester und die wenigstens eine Metallseife und/oder Seife mit einem Gesamtanteil von 33 bis 90 Gew.-% enthalten sind.

Ferner betrifft die Erfindung einen Stift umfassend eine solche Mine und ein Verfahren zur Herstellung einer solchen Mine oder Kreide.

## Beschreibung

Die Erfindung betrifft eine Mine oder Kreide für Schreib-, Mal- und/oder Kosmetikzwecke sowie einen Stift mit einer solchen Mine. Ferner betrifft die Erfindung ein Verfahren zur Herstellung einer Mine oder Kreide.

Thermoplastisch herstellbare, wasserfeste Minen unter Verwendung von Polystyrol, Polyvinylbutyral oder anderen Thermoplasten als Bindemittel sind bekannt. Solche Minen weisen zwar eine ausreichende Festigkeit auf, sind aber hinsichtlich ihres Aufstrichverhaltens nicht optimal, da beim Erzeugen eines Aufstriches relativ hohe Kräfte erforderlich sind, um eine ausreichende Decckraft zu erreichen. In der EP 2 520 442 B1 sind beispielsweise Farbstiftminen bzw. Farbkreiden beschrieben deren Zusammensetzung neben Füllstoffen und Farbmitteln auf Fetten und Wachsen basiert, wobei diese mit einem Anteil von maximal 30 Gew.-% enthalten sind. Ferner existieren thermoplastisch herstellbare, wasserlösliche, aquarellierbare Farbstiftminen, welche Zucker beinhalten und beispielsweise in EP 1 552 958 B1 oder EP 3 272 819 B1 beschrieben sind. Diese Minen zeigen eine gute mechanische Festigkeit und lassen sich gut mit Durchmessern zwischen 2,8 bis 6 mm fertigen. Auch die Herstellung von Kreiden mit größeren Durchmessern als 6 mm ist denkbar.

Nachteilig bei den genannten Minen ist, dass insbesondere bei wasserfesten Minen Kunststoffe als Bindemittel und/oder Wachsbestandteile verwendet werden, die petrochemische Polymere, die mindestens teilweise nicht biologisch abbaubar sind, enthalten. Außerdem ist es bei den zuvor genannten Minen nicht möglich, sehr weich applizierbare Minen herzustellen.

Es ist daher Aufgabe der Erfindung eine Mine oder Kreide für Schreib-, Mal- und/oder Kosmetikzwecke anzugeben, die sowohl wasserlöslich als auch wasserfest eingestellt werden kann und hinsichtlich des enthaltenen Bindemittels verbessert ist. Ferner ist es Aufgabe der Erfindung einen Stift mit einer Mine sowie ein Verfahren zur Herstellung einer Mine oder Kreide anzugeben, welches eine kostengünstige und effiziente Herstellung der Minen oder Kreiden erlaubt.

Die Aufgabe wird gelöst mit einer biologisch abbaubaren und/oder kompostierbaren Mine oder Kreide für Schreib-, Mal- und/oder Kosmetikzwecke, insbesondere einer Farbmine oder Farbkreide, mit den Merkmalen gemäß Anspruch 1. Danach sind in der Mine oder Kreide wenigstens ein Fettsäureester, wenigstens eine Metallseife, also ein Metallsalz einer Fett-, einer Harz- oder Naphthensäure, und/oder Seife, also ein wasserlösliches Fettsäuresalz der Natrium- und Kalium-Salze, und wenigstens ein Farbmittel enthalten. Erfindungsgemäß sind der wenigstens eine Fettsäureester und die wenigstens eine Metallseife und/oder Seife mit einem Anteil von 33 bis 90 Gew.-%, insbesondere mit einem Anteil von mindestens 35°Gew.-% und/oder mit einem Anteil von maximal 80 Gew.-%, in der Mine oder Kreide enthalten.

Mit anderen Worten: In der Mine oder Kreide werden Fettsäureester und Metallsalze kombiniert. Der vorstehend angegebene Gehaltsbereich bezieht sich dabei auf den Gesamtanteil der insgesamt in der Mine oder Kreide enthaltenen Fettsäureester, Metallseife(n) und/oder Seife(n) bezogen auf die gesamte Mine oder Kreide.

Vorzugsweise ist der wenigstens eine Fettsäuereester mit einem Anteil von 2 bis 30 Gew.-% enthalten, die wenigstens eine Metallseife und/oder Seife mit einem Anteil von 10 bis 70 Gew.-%.

Unter einer biologisch abbaubaren und/oder kompostierbaren Mine oder Kreide ist vorliegend eine Mine oder Kreide zu verstehen, bei der kein Bindemittel enthalten ist, welches nicht in einer festgeschriebenen Zeit unter definierten Temperatur-, Sauerstoff- und Feuchtebedingungen in der Anwesenheit von Mikroorganismen oder Pilzen zu mehr als 90 Prozent zu Wasser, CO2 und Biomasse abgebaut haben muss. Vorliegend ist als Bindemittel wenigstens ein Fettsäureester, wenigstens eine Metallseife und/oder Seife enthalten. Im Folgenden wird vereinfachend vorwiegend auf eine Mine Bezug genommen, die Ausführungen gelten jedoch ebenso auch für eine Kreide.

Die Mine oder Kreide ist aus einer die genannten Komponenten bzw. gegebenenfalls weitere Ausgangsstoffe, enthaltenen Minengrundmasse bzw. Minen-Ausgangsmasse hergestellt, die thermoplastisch verarbeitet und extrudiert wird, um die Ausgangsstoffe bei erhöhter Temperatur aufzuschmelzen und zu binden. Ebenso kann die Mine aus solchen Ausgangsstoffen hergestellt sein, welche während der thermoplastischen Verarbeitung miteinander chemisch reagieren, um eine Minenmasse bzw. Mine enthaltend Fettsäureester und Metallseifen und/oder Seifen zu erhalten. In diesem Fall findet die Verseifung somit während der thermoplastischen Verarbeitung statt. Gegebenenfalls bei der Herstellung der Mine zur Förderung der Durchmischung der Ausgangsstoffe zugesetztes Wasser oder bei der chemischen Reaktion entstehendes Wasser wird anschließend wieder entfernt, insbesondere vollständig entfernt, sodass der Anteil an freiem Wasser auf maximal 0,5 Gew.-%, insbesondere auf 0 Gew.-% reduziert ist. Die Minenmasse sowie die Mine selbst sind somit wasserfrei und enthalten höchstens 0,5 Gew.-% Wasser. Mittels Extrusion werden Minen- oder Kreidestränge ausgeformt und die Minen- oder Kreidestränge werden zu Minen oder Kreiden abgelängt. Nach Erkalten der Minenmasse liegt bereits die fertige Mine oder Kreide vor, ein aufwändiges Entfernen von Wasser oder Lösungsmitteln ist nicht erforderlich.

Die Verwendung von Fettsäuren oder Fettsäureestern und Metallsalzen von Fettsäuren in extrudierten Farbstiftminen ist durchaus bekannt. Üblicherweise werden bisher in wasserfesten Minen die Fettsäuren als niedrigschmelzendes Wachs für eine bessere Minenabgabe beim Schreiben oder Malen zugegeben, die Metallsalze in geringen Mengen (≤ 12 %) als Geliermittel. Bei wasserlöslichen extrudierten Minen wirkt die Stärke als Verdicker, Fettsäuren dienen als niedrigschmelzendes Wachs für eine bessere Abgabe und Seife wird hier etwa zu 50% des Bindemittels zugegeben, um die Wasserlöslichkeit zu verbessern.

Nach einer Vielzahl von Versuchen konnte nun überraschenderweise festgestellt werden, dass Metallseifen und/oder Seifen in größeren Gehaltsmengen in Minen und Kreiden als Bindemittel fungieren können und zudem einen Weg eröffnen, sowohl wasserfeste als auch wasserlösliche Minen in einem thermoplastischen Verfahren herzustellen. Die Minen weisen eine gute mechanische Festigkeit auf und zeigen ein gutes Applikationsverhalten. Darüber hinaus können sie kostengünstig und effizient in einem thermoplastischen Prozess hergestellt werden. Durch die enthaltene Metallseife und/oder Seife bzw. die Verseifung während der Herstellung wird ferner eine Verbesserung der Farbabgabe bei wenig Druck und eine bessere Übermalbarkeit erzielt.

Insbesondere sind wenigstens ein Fettsäureester und/oder wenigstens eine Metallseife(n) und/oder Seife(n) basierend auf natürlichen Fetten, Wachsen und/oder Ölen enthalten, die aus einem Gemisch verschiedener Carbonsäuren bestehen, die sich in Kettenlänge, Anzahl, Verteilung und Richtung der Doppelbindungen unterscheiden. Zur Herstellung von biologisch abbaubarer und/oder kompostierbarer, biobasierter Minen oder Kreiden werden vorzugsweise natürliche Fettsäuren verwendet. Je länger die Kette der Fettsäuren, desto höher ist deren Schmelzpunkt und die Bruchkraft, aber desto langsamer läuft eine Verseifungsreaktion während der Herstellung ab und desto weniger Abgabe gibt es beim Schreiben und Malen. Je mehr Doppelbindungen die Fettsäure aufweist, desto niedriger ist deren Schmelzpunkt und die Bruchkraft nimmt ab. Die Abgabe auf der Schreib- oder Malunterlage erhöht sich hingegen. Für Minen gut geeignet sind Kombinationen aus kurzkettigen Fettsäuren oder Fettsäuren mit Doppelbindungen für die Abgabe und langkettige Fettsäuren für die Stabilität, wobei ein Bereich von Kettenlängen C8 (aus Kokosfett) bis C30 (aus Bienenwachs) empfohlen ist. Gegebenenfalls kann wenigstens ein Fettsäureester und/oder wenigstens eine Metallseife und/oder Seife basierend auf einer Harzsäure und/oder einer Naphthensäure und/oder einem polaren Wachs enthalten sein, wobei deren gesamter Anteil auf maximal 5 Gew.-% begrenzt ist, um die biologische Abbaubarkeit der Mine zu erhalten.

Vorzugsweise ist wenigstens ein Fettsäureester und/oder wenigstens eine Metallseife und/oder Seife basierend auf einer Carbonsäure, insbesondere einer Fettsäure wie Stearinsäure, Palmitinsäure, Ölsäure, Laurinsäure, Myristinsäure, Caprinsäure, Palmitoleinsäure, Linolsäure, Reiskleiewachs, Sonnenblumenwachs enthalten.

Des Weiteren basiert die wenigstens eine Metallseife und/oder Seife insbesondere auf Metallionen einwertiger Salze der Alkalimetalle, insbesondere Lithium, Natrium und/oder Kalium und/oder auf Metallionen zweiwertiger Erdalkalimetalle, insbesondere Magnesium, Kalzium und Barium und/oder auf Metallionen von Metallen, insbesondere Aluminium und Zink, enthalten. Eine Kombination verschiedener Metallionen ist ebenso möglich wie der Einsatz anderer anionischer oder kationischer Tenside, wie z.B. Natriumcocoylisethionat.

Minen oder Kreiden können vorzugsweise eine Seife basierend auf einer Fettsäure, insbesondere einer Fettsäure bis maximal C16 und/oder einer Mischung aus längerkettigen Fettsäuren als C18, wie z.B. Stearinsäure, und Fettsäuren bis maximal C16, und basierend auf Natrium und/oder Kalium als Metallion enthalten. Durch Fettsäuren bis maximal C16 ist die Mine oder Kreide wasserlöslich. Eine Verbesserung der Wasserlöslichkeit kann zudem durch Erhöhung der Natrium- oder Kaliumionen bzw. des Anteils der Seife im Vergleich zu der Fettsäure erreicht werden.

Gemäß einer weiteren vorteilhaften Ausbildung enthält die Mine oder Kreide eine Metallseife basierend auf den Kationen Lithium, Magnesium, Kalzium, Barium, Aluminium und Zink als Metallion und/oder basierend auf Natrium und/oder Kalium als Metallion in Kombination mit einer längerkettigen Fettsäure als C18, z.B. Stearinsäure. Dadurch ist die Mine oder Kreide wasserfest. Auch die Verwendung von Kalium- und Natriumionen innerhalb der wasserfesten Rezeptur ist möglich, jedoch nur in Kombination mit langkettigen Fettsäuren C18 oder längerkettigen Fettsäuren. Auch die Verwendung von kürzerkettigen Fettsäuren ist möglich, jedoch nur in geringem Verhältnis zu den längerkettigen Fettsäuren, abhängig von der tatsächlichen Kettenlänge. Eine Reduzierung des Anteils an Kationen führt außerdem auch zu einer Reduzierung der Wasserlöslichkeit der Minen und begünstigt damit eine Wasserfestigkeit.

Die für die Herstellung benötigten Metallsalze von Fettsäuren können entweder als Seife zur Minenherstellung eingesetzt werden. Es ist jedoch auch möglich, eine Verseifung einer Fettsäure mit einem Metallhydroxid oder Metallsalz, z.B. Alkali-, Erdalkali- oder Metall-Carbonate oder -phosphate, im Herstellprozess durchzuführen.

Gemäß einer vorteilhaften Ausgestaltung liegt ein Masseverhältnis der Fettsäure(n) zu Metallseife(n) und/oder Seife(n) in der Mine oder Kreide zwischen 1:1 bis 1:10. Mit anderen Worten: Der Anteil an Fettsäure(n) zu Metallseife(n) und/oder Seife(n) ist derart gewählt, dass die Mine oder Kreide eine Überfettung zwischen 10 und 50% aufweist. Die Fettsäuren und Metallseife(n) und/oder Seife(n) oder die zur Verseifung dienenden Metallhydroxide werden also so kombiniert, dass ein Überschuss an Fettsäuren (Überfettung) < 50% erreicht wird.

Je geringer die Überfettung, desto höher ist der Anteil an Metallionen im Vergleich zu Fettsäure(n) und der Abstrich wird weniger gleitend. Auf glattem Untergrund zeigt sich auch ohne Druck eine deutlich bessere Abgabe. Zudem wird die Übermalbarkeit verbessert. Eine Überfettung kleiner 10% ist nicht zielführend, da die Bruchkraft hier deutlich abnimmt. Eine geringere Überfettung führt dazu, dass der Schmelzpunkt der Ausgangsstoffe bei der Herstellung erhöht wird und die Schmelze eine höhere Viskosität aufweist. Dadurch lässt sich eine bessere Formstabilität der Minen bei hohen Temperaturen, also z.B. beim Schneiden bzw. Ablängen des Minenstrangs nach der Extrusion, oder bei einer potentiellen Lagerung des Produkts bei hohen Temperaturen, z.B. in einem Überseecontainer oder ähnlichem, erreichen.

Bei einer bevorzugten Weiterbildung ist das Farbmittel mit einem Anteil von 0,1 bis 35 Gew.-%, insbesondere mit einem Anteil von 0,1 bis 25 Gew.-% in der Mine oder Kreide enthalten.

Vorzugsweise sind als Farbmittel Farbstoffe und/oder organische Pigmente und/oder anorganische Pigmente enthalten, insbesondere feinpulverisierte organische und/oder anorganische Pigmente mit einer Korngrößenverteilung D90 < 100 µm. Typische organische Pigmente sind solche aus der Gruppe der Phthalocyanin Pigmente, Azopigmente, Indanthron Pigmente, Perylen Pigmente, Diketopyrrolopyrrol-Pigmente (DPP). Als anorganische Pigmente sind Eisenoxide, Titandioxid, Manganviolett, Ultramarin-Blau, Miloriblau sowie Ruß und andere Farbmittel denkbar. Minen oder Kreiden, deren Pigmente eine solche Korngrößenverteilung aufweisen, zeigen aufgrund des hohen Anteils an Metallseife(n) und/oder Seife(n) in der vorliegenden Mine einen Abstrich mit guter Farbintensität ohne jedoch beim Abstreichen zu Kratzen oder ungleichmäßig in der Farbe abzugeben, wie dies typischerweise bei gröberen Pigmenten auftreten würde. Die Korngröße der Pigmente lässt sich mit verschiedenen Techniken ermitteln, die dem Fachmann bekannt sind. Beispielsweise können Korngröße und Korngrößenverteilung der Pigmente mittels Laserbeugung bestimmt werden. Vorliegend wurde zur Bestimmung der Korngröße und Korngrößenverteilung das Gerät Mastersizer 3000 der Firma Malvern Panalytical eingesetzt.

In der Regel werden bei klassischen Farbstiftminen Füllstoffe eingesetzt. Durch die Kombination aus Fettsäuren und Metallseifen und/oder Seifen als Bindemittel ist es grundsätzlich möglich, eine füllstofffreie Mine zu erzeugen. Es können jedoch ein oder mehrere Füllstoffe enthalten sein, um das Eigenschaftsprofil der Mine oder Kreide, insbesondere das Abstrichverhalten und die Bruchkraft, zu beeinflussen. Dabei hat sich die Zugabe eines Füllstoffes mit einem maximalen Anteil von 75 Gew.-% in der Mine oder Kreide als vorteilhaft erwiesen. Füllstoffe sind meist natürliche Rohstoffe wie anorganische Mineralien (Kaolin, CaCO₃, Ton, Quarzmehl, Talkum, Mica) oder Pflanzenfasern (Holzspäne, Cellulose). Die Bruchkraft der Mine erreicht je nach Füllstoffgehalt ein Maximum und fällt, wenn der Bindemittelgehalt, also der Anteil an Fettsäureester(n) und Metallseifen und/oder Seifen nicht mehr ausreicht die Festkörper, also die nicht schmelzbaren Stoffe wie Füllstoffe, Pigmente oder auch mineralische Additive ausreichend zu binden, wieder ab.

Zudem können Additive eingesetzt werden, welche zum einen als Hilfsmittel für die Verarbeitung dienen. Ferner wird es durch den Einsatz von Additiven teilweise möglich, die Wasserfestigkeit bzw. Wasserlöslichkeit innerhalb der Minenzusammensetzung zu steuern. Gemäß einer vorteilhaften Weiterbildung ist in der Mine oder Kreide wenigstens ein Additiv enthalten. Der maximale Anteil an Additiven beträgt insbesondere 15 Gew.-%. Als mögliche Additive können Hydroxyle, z. B. Glycerin oder Zucker, aber auch Carbonsäuren, wie Milchsäure, Zitronensäure, Essigsäure und/oder Salze enthalten sein. Diese Additive können Bruchkraft, Abstrichverhalten, Farbton sowie Wasserlöslichkeit der Mine oder Kreide beeinflussen.

Hydroxyle werden nach der Anzahl der enthaltenen Hydroxylgruppen in Alkohole und Polyole unterschieden. Mehrere Hydroxylgruppen, beispielsweise bei Stärke oder Cellulose, erlauben eine Vernetzung der Hydroxyle und der Fettsäuren, insbesondere Carbonsäuren. Durch den Einsatz solcher Hydroxyle nehmen die Bruchkraft und Durchbiegung der Minen zu, der Abstrich wird etwas bremsiger. Neben der Anzahl der Hydroxylgruppen ist auch die Kettenlänge relevant. Langkettige Hydroxyle, wie z.B. im Sonnenblumenwachs (C26-C32) erhöhen die Bruchkraft, den Schmelzpunkt und die Wasserfestigkeit, die Reaktivität und biologische Abbaubarkeit nehmen hingegen ab. Der Abstrich wird gleitender.

Die Verwendung spezieller Carbonsäuren, wie Essigsäure, Milchsäure oder Zitronensäure als Additiv ist ebenso denkbar. Zitronensäure ist beispielsweise besonders kurzkettig und damit bereits in kleinen Mengen von bis zu 0,5 Gew.-% als schneller Reaktionspartner geeignet. Zitronensäure hat mehrere Säuregruppen und kann somit als Vernetzungsadditiv verwendet werden, wodurch die Härte der Mine erhöht wird. Milchsäure hat sowohl eine Carboxyl- als auch eine Hydroxylgruppe, wodurch diese miteinander verestern können.

Durch den Einsatz von Salzen kann die Wasserlöslichkeit, Hygroskopie oder Härte der Mine oder Kreide beeinflusst werden.

Die Zusammensetzung der erfindungsgemäßen Minen oder Kreiden kann somit wie folgt angegeben werden:

| | |
|---|---|
| Fettsäureester und Metallseife(n) und/oder Seife(n) | 33 bis 90 Gew.-% |
| Füllstoffe | 0 bis 75 Gew.-% |
| Farbmittel | 0,1 bis 35 Gew.-% |
| Additive | 0 bis 15 Gew.-% |

Die Aufgabe wird ferner gelöst mit einem Stift mit den Merkmalen gemäß Anspruch 12, welcher eine Mine mit den vorstehend beschriebenen Merkmalen umfasst. Der Stift weist dabei insbesondere eine Ummantelung aus Holz oder aus einem Holzersatzwerkstoff auf, welcher die Mine umhüllt. Als Holzersatzwerkstoff wird anstelle von natürlichem Holz beispielsweise ein sogenanntes Wood-Plastic-Compound eingesetzt, sodass der Stift beispielsweise in einfacher Weise durch Co-Extrusion der Mine und der Ummantelung hergestellt werden kann.

Des Weiteren wird die Aufgabe gelöst durch ein Verfahren zur Herstellung einer biologisch abbaubaren und/oder kompostierbaren Mine oder einer Kreide mit den Merkmalen gemäß Anspruch 13. Gemäß dem Verfahren werden die zur Herstellung der Mine erforderlichen Ausgangsstoffe bzw. die in der Minen-Ausgangsmasse enthaltenen Stoffe, also der wenigstens eine Fettsäureester, die wenigstens eine Metallseife und/oder Seife oder die zur Metallseife und/oder Seife reagierenden Metallhydroxide und/oder Metallsalze und insbesondere wenigstens ein weiterer Fettsäureester, und das wenigstens eine Farbmittel bei erhöhter, ein Schmelzen der Ausgangsstoffe bewirkenden Temperatur thermoplastisch verarbeitet bzw. vermischt und/oder homogenisiert und miteinander verbunden. Gegebenenfalls werden weitere Stoffe, wie beispielsweise Füllstoffe oder Additive beigefügt und gemeinsam mit den vorstehend genannten Ausgangsstoffen thermoplastisch verarbeitet. Die daraus erhaltene Minengrundmasse wird zu Minen oder Kreidesträngen extrudiert. Der wenigstens eine Fettsäureester, die wenigstens eine Metallseife und/oder Seife oder die zur Metallseife und/oder Seife reagierenden Fettsäuren und Metall hydroxide und/oder Metallsalze werden derart zugegeben, dass der wenigstens eine Fettsäureester und die wenigstens eine Metallseife und/oder Seife mit einem Anteil von 33 bis 90 Gew.-% in der Mine oder Kreide enthalten sind.

Als Rahmenrezeptur für die Mine bzw. zur Herstellung der Mine erforderlichen Ausgangsstoffe bzw. die in der Minen-Ausgangsmasse enthaltenen Stoffe kann angegeben werden:

| | |
|---|---|
| Bindemittel Fettsäuren + Metallionen / Metallhydroxide oder Metallsalze von Fettsäuren | 33 bis 90 Gew.-% |
| Füllstoffe | 0 bis 75 Gew.-% |
| Farbmittel | 0,1 bis 35 Gew.-% |
| Additive | 0 bis 15 Gew.-% |

Mit anderen Worten: Bei der Herstellung können ein Fettsäureester und bereits als solche vorliegende Metallseifen, also Fettsäuresalze, als Ausgangsstoffe verwendet werden. Sofern die Metallseifen und/oder Seifen noch nicht als solche vorliegen, werden zur Herstellung der Mine oder Kreide wenigstens ein Fettsäureester sowie zur Metallseife und/oder Seife reagierende Metallhydroxide und/oder Metallsalze und gegebenenfalls weitere Fettsäureester zugegeben und eine Verseifung des Fettsäureesters findet während des Herstellprozesses statt.

Die Herstellung der Mine oder Kreide kann im Nass- oder Trockenverfahren erfolgen.

Vorzugsweise wird den Ausgangsstoffen Wasser, insbesondere mit einem Anteil von etwa 5 bis 20 Gew.-% zugegeben, welches gemeinsam mit der wenigstens einen Fettsäure, der wenigstens einen Metallseife und/oder Seife oder der zur Metallseife und/oder Seife reagierenden Fettsäuren und Metallhydroxide, und dem wenigstens einen Farbmittel thermoplastisch verarbeitet wird, wobei der Anteil des freien Wassers während des Mischens auf höchstens 0,5 Gew.-%, vorzugsweise auf 0 Gew.-% reduziert wird ("Nassverfahren"). Die extrudierte oder ausgeformte Minengrundmasse weist durch die Reduzierung des Wassergehaltes einen geringen Feuchtigkeitsgehalt auf, sodass nach dem Erkalten der Minengrundmasse bereits die fertige Mine vorliegt. Ein aufwändiges Entfernen von Wasser oder sonstigen Lösungsmitteln entfällt.

Vorzugsweise werden der wenigstens eine Fettsäureester, die wenigstens eine Metallseife und/oder Seife oder zur Metallseife und/oder Seife reagierende Metallhydroxide und/oder Metallsalze und insbesondere der wenigstens eine weitere Fettsäureester, und das wenigstens eine Farbmittel bei einer Temperatur von mindestens 50°C thermoplastisch verarbeitet.

Als Fettsäureester und/oder Metallseifen und/oder Seifen werden vorzugsweise solche basierend auf natürlichen Fetten, Wachsen und/oder Ölen eingesetzt.

Vorzugsweise werden ein Fettsäureester und/oder eine Metallseife oder eine Seife basierend auf einer Carbonsäure, insbesondere einer Fettsäure wie Stearinsäure, Palmitinsäure, Ölsäure, Laurinsäure, Myristinsäure, Caprinsäure, Palmitoleinsäure, Linolsäure, Reiskleiewachs, Sonnenblumenwachs eingesetzt und/oder eine Metallseife oder Seife basierend auf Metallionen einwertiger Salze der Alkalimetalle, insbesondere Lithium, Natrium und/oder Kalium und/oder basierend auf Metallionen zweiwertiger Erdalkalimetalle, insbesondere Magnesium, Kalzium und Barium und/oder basierend auf Metallionen von Metallen, insbesondere Aluminium und Zink eingesetzt.

Als Metallhydroxide werden insbesondere Lithium-, Natrium-, Magnesium-, Kalzium-, Barium-, Aluminium- und Zinkhydroxide eingesetzt.

Um wasserlösliche Minen oder Kreiden herzustellen, werden vorzugsweise eine Seife basierend auf einer Fettsäure, insbesondere einer Fettsäure bis maximal C16 und/oder einer Mischung aus längerkettigen Fettsäuren als C18 und Fettsäuren bis maximal C16, und basierend auf Natrium und/oder Kalium als Metallion, oder eine Fettsäure, insbesondere einer Fettsäure bis maximal C16 und/oder einer Mischung aus längerkettigen Fettsäuren als C18 und kurzkettigen Fettsäuren bis maximal C16 und Natriumhydroxide und/oder Kaliumhydroxide und/oder Kaliumcarbonate zugegeben.

Um wasserfeste Minen oder Kreiden herzustellen, werden vorzugsweise eine Metallseife basierend auf Lithium, Magnesium, Kalzium, Barium, Aluminium und/oder Zink als Metallion und/oder basierend auf Natrium und/oder Kalium als Metallion in Kombination mit einer längerkettigen Fettsäure als C18 oder Lithium-, Magnesium-, Kalzium-, Barium-, Aluminium- und Zinkhydroxide und/oder Natriumhydroxide und/oder Kaliumhydroxide in Kombination mit einer längerkettigen Fettsäure als C18 zugegeben.

Die Fettsäure(n) und Metallseife(n) und/oder Seife(n) und/oder Metallhydroxide werden insbesondere derart zugegeben, dass das Verhältnis von Fettsäure(n) zu Metallseife(n) und/oder Seife(n) in der Mine oder Kreide zwischen 1:10 bis 10:10 liegt, um eine Überfettung zwischen 10 und 50% zu erreichen.

Vorzugsweise wird das Farbmittel derart zugegeben, dass dieses mit einem Anteil von 0,1 bis 35 Gew.-%, insbesondere mit einem Anteil von 0,1 bis 25 Gew.-% in der Mine enthalten ist. Als Farbmittel können Farbstoffe und/oder organische Pigmente und/oder anorganische Pigmente zugegeben werden, insbesondere organische und/oder anorganische Pigmente mit einer Korngrößenverteilung D90 < 100 µm.

Ferner kann wenigstens ein Füllstoff, vorzugsweise Calciumcarbonat, Kaolin, Talkum, Bimsmehl, Quarzmehl, Mica, Weißpigmente und/oder Pflanzenfasern, zugegeben werden, insbesondere derart, dass dieser mit einem maximalen Anteil von 75 Gew.-% in der Mine oder Kreide enthalten ist.

Gemäß einer vorteilhaften Weiterbildung wird wenigstens ein Additiv, vorzugsweise ausgewählt aus der Gruppe Hydroxyle und/oder Carbonsäuren und/oder Salze, zugegeben, insbesondere derart, dass dieses mit einem maximalen Anteil von 15 Gew.-% enthalten ist.

Nachfolgend sind sieben Beispielrezepturen für eine biologisch abbaubare und/oder kompostierbare Mine oder Kreide aufgeführt. Die Herstellung solcher Minen kann beispielsweise im sogenannten "Nassverfahren" erfolgen. Hierbei werden trockene Rohmaterialien bzw. Ausgangsstoffe in einen Schnellmischer oder Kneter gegeben und während des Mischvorganges homogenisiert. Anschließend erfolgt die Zugabe von Wasser, insbesondere mit einem Anteil von 5 bis 20 Gew.-%. Durch ein nachfolgendes Aufheizen auf Temperaturen über 100°C, vorzugsweise bei reduzierter Drehzahl, werden die wasserlöslichen Bestandteile aufgelöst. Anschließend kann durch Öffnen des Mischers oder Kneters eine weitere Trocknung der Masse vorgenommen werden. Während des Verfahrens findet eine Verseifung der zugegebenen Metallhydroxide und Fettsäuren statt.

Alternativ ist die Herstellung der Mine in einem sogenannten "Trockenverfahren" möglich. Dies ist insbesondere dann zu empfehlen, wenn bereits die Metallseifen und/oder Seifen, also die Fettsäuresalze vorliegen und darüber hinaus nur schmelzbare Materialien als Additive verwendet werden, wie z. B. Sorbitol oder Dextrin. Hierzu werden alle Rohmaterialien bzw. Ausgangsstoffe in einem Schnellmischer oder Extruder gegeben. Die Aufheizphase erfolgt bis zum Schmelzpunkt bei Temperaturen über 100 °C unter Verwendung hoher Drehzahlen. Anschließend entweicht in einer "Trockenphase" des Mischvorgangs bei unterhalb der Schmelztemperatur die Restfeuchte der Materialien.

Sowohl beim Nass- als auch beim Trockenverfahren wird das erhaltene Granulat anschließend abgekühlt und zu Minen- oder Kreidensträngen extrudiert, beispielsweise auf ein oder bevorzugt zwei Schneckenextrudern oder Kolbenextrudern. Anschließend werden die Minen- oder Kreidenstränge abgelängt.

### Beispiel 1: Rote wasserlösliche Mine im Nassverfahren (Verseifung beim Mischen)

### Ausgangsrezeptur:

| | |
|---|---|
| Stearin | 24 Gew.-% |
| Rapsöl | 12 Gew.-% |
| Kaliumhydroxid | 4 Gew.-% |
| Kaolin | 55 Gew.-% |
| Lithopone DS 30 | 3 Gew.-% |
| Pigment 1 (Pigment-rot 254 5 CI 56110) | 1 Gew.-% |
| Pigment 2 (Pigment Red 122) | 1 Gew.-% |

Eine Mine oder Kreide bzw. ein Stift mit einer Mine mit einer Zusammensetzung entsprechend Beispiel 1 wird im Nassverfahren hergestellt. Es werden zusätzlich 15 Gew.-% Wasser zugegeben, die bis zum Ende des Mischverfahrens vollständig wieder verdampfen. Im Mischprozess reagiert das Kaliumhydroxid mit Rapsöl und Stearin zu Kaliumseifen. Dabei wird Glycerin frei.

Reaktionsgleichung (R1, R2, R3 = Fettsäure):

| 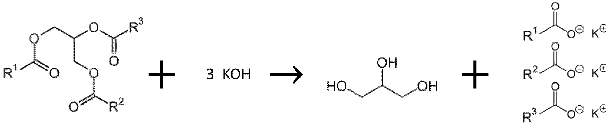 | | | |
|---|---|---|---|
| Fettsäureester | 3 Kaliumhydroxid | Glycerin | 3 Fettsäureseifen |

Natürliches Rapsöl setzt sich aus verschiedenen mit Glycerin (ca. 5%) veresterten Fettsäuren, wie Ölsäure (ca. 59%), Linolsäure (ca. 21%), Linolensäure (ca. 8%), Palmitinsäure (ca. 6%) und Stearinsäure (ca. 1%) zusammen. Diese unterscheiden sich durch Kettenlänge und Anzahl der Doppelbindungen. Aufgrund der Reaktivität verseifen kürzerkettige Fettsäuren leichter als längerkettige, sowie bei Fettsäuren mit gleicher Kettenlänge ungesättigte Fettsäuren leichter als gesättigte (mit Doppelbindungen).

Nach der Reaktion setzt sich die Minenmasse und die Kreiden bzw. Minen folgendermaßen zusammen:

| | |
|---|---|
| Stearin | 15 Gew.-% |
| Kaliumstearat | 9,9 Gew.-% |
| Kaliumoleat | 8,1 Gew.-% |
| Kaliumlinolat | 2,8 Gew.-% |
| Kaliumlinolenat | 1,1 Gew.-% |
| Kaliumpalmitat | 0,9 Gew.-% |
| Glycerin | 2,2 Gew.-% |
| Kaolin | 55 Gew.-% |
| Lithopone DS 30 | 3 Gew.-% |
| Pigment 1 (Pigment-rot 254 5 CI 56110) | 1 Gew.-% |
| Pigment 2 (Pigment Red 122) | 1 Gew.-% |

Durch die Verseifung mit Kalium wird die Mine sehr weich in der Abgabe und haftet auch auf glatten Untergründen.

### Beispiel 2: grüne wasserlösliche Mine im Nassverfahren (Verseifung beim Mischen)

### Ausgangsrezeptur:

| | |
|---|---|
| Stearin | 20 Gew.-% |
| Palmöl | 12 Gew.-% |
| Kaliumcarbonat | 5 Gew.-% |
| Kaolin | 58 Gew.-% |
| Titandioxid | 2 Gew.-% |
| Pigment (Pigment Grün 7) | 3 Gew.-% |

Eine Mine oder Kreide bzw. ein Stift mit einer Mine mit einer Zusammensetzung entsprechend Beispiel 2 wird im Nassverfahren hergestellt. Es werden zusätzlich 20 Gew.-% Wasser zugegeben, die bis zum Ende des Mischverfahrens wieder verdampfen. Im Mischprozess reagiert das Kaliumcarbonat mit Palmöl und Stearin zu Kaliumseifen. Dabei werden Glycerin und Kohlendioxid frei. Das Kohlendioxid entweicht.

Reaktionsgleichung (R1, R2, R3 = Fettsäure):

| 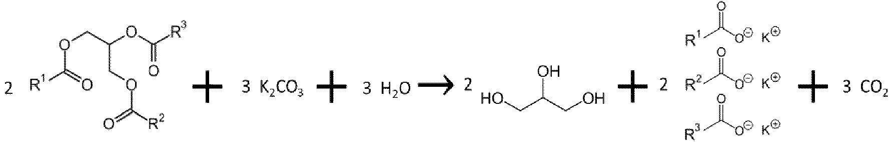 | | | | | |
|---|---|---|---|---|---|
| 2 Fettsäureester | 3 Kaliumcarbonat | 3 Wasser | 2 Glycerin | 6 Fettsäureseifen | 3 Kohlendioxid |

Kaliumcarbonat ist im Vergleich zum Kaliumhydroxid nicht ätzend eingestuft. Natürliches Palmöl setzt sich aus verschiedenen mit Glycerin (ca. 4,9%) veresterten Fettsäuren wie Laurinsäure (ca. 0,5%), Myristinsäure (ca. 0,9%), Palmitinsäure (ca. 41,8%), Ölsäure (ca. 38,1%), Linolsäure (ca. 9,0%) und Stearinsäure (ca. 4,8%) zusammen.

Nach der Reaktion setzt sich die Minenmasse und die Kreiden bzw. Minen folgendermaßen zusammen:

| | |
|---|---|
| Stearin | 11,3 Gew.-% |
| Kaliumstearat | 10,4 Gew.-% |
| Kaliumoleat | 5,3 Gew.-% |
| Kaliumpalmitat | 5,8 Gew.-% |
| Kaliumlinolat | 1,2 Gew.-% |
| Kaliummyristat | 0,1 Gew.-% |
| Kaliumlaurat | 0,1 Gew.-% |
| Glycerin | 2,3 Gew.-% |
| Kaolin | 58,6 Gew.-% |
| Titandioxid | 2 Gew.-% |
| Pigment (Pigment Grün 7) | 3 Gew.-% |

Die Kombination der Fettsäureseifen sorgt für eine besonders gute Wasserlöslichkeit bei guter Stabilität der Mine.

### Beispiel 3: blaue, wasserfeste Buntstiftmine im Trockenverfahren (Rohstoff bereits verseift)

Da die Seife bereits vorliegt entspricht bei dieser Rezeptur die Ausgangsrezeptur der Minenrezeptur und setzt sich folgendermaßen zusammen:

| | |
|---|---|
| Reiskleiewachs | 5 Gew.-% |
| Natriumstearat | 30 Gew.-% |
| Kaolin | 60 Gew.-% |
| Pigment (Pigment blue 15:1) | 5 Gew.-% |

Eine Mine oder Kreide bzw. ein Stift mit einer Mine mit einer Zusammensetzung entsprechend Beispiel 3 wird im Trockenverfahren hergestellt. Die Mine gibt auch bei geringem Anpressdruck oder beschichtetem Papier weich und farbintensiv ab.

### Beispiel 4: Pigmentfreie blaue Buntstiftmine mit Farbstoff im Nassverfahren (Rohstoffe bereits verseift)

| | |
|---|---|
| Stearin | 7 Gew.-% |
| Natriumstearat | 42 Gew.-% |
| Sorbitol | 7 Gew.-% |
| Kaolin | 43,8 Gew.-% |
| Farbstoff (Brilliant Blue E 133) | 0,2 Gew.-% |

Eine Mine oder Kreide bzw. ein Stift mit einer Mine mit einer Zusammensetzung entsprechend Beispiel 4 wird im Nassverfahren hergestellt. Es werden zusätzlich 5 Gew.-% Wasser zugegeben, die bis zum Ende des Mischverfahrens vollständig wieder verdampfen. Der Farbstoff wird zu Beginn in dem zugegebenen Wasser gelöst und dadurch gleichmäßig verteilt. Die Mine ist durch den Farbstoff und geringen Füllstoffgehalt besonders leuchtend.

### Beispiel 5: Gelbe wasserfeste Mine mit hohem Wachsanteil im Trockenverfahren (Rohstoffe bereits verseift)

| | |
|---|---|
| Stearin | 15 Gew.-% |
| Hydriertes Sojaöl | 4 Gew.-% |
| Mikrokristallines Wachs | 1 Gew.-% |
| Kalziumstearat | 20 Gew.-% |
| Sorbitol | 15 Gew.-% |
| Kaolin | 40 Gew.-% |
| Pigment Yellow 1 | 5 Gew.-% |

Diese Mine kann zum Markieren verwendet werden, da sie weich abgibt und Schrift nicht überdeckt. Das mikrokristalline Wachs ist lediglich mit einem Anteil von 1 Gew.-% enthalten und steht somit der biologischen Abbaubarkeit des Bindemittels der Mine nicht entgegen.

### Beispiel 6: grüne wasserfeste Softpastellkreide im Trockenverfahren (Rohstoffe bereits verseift)

| | |
|---|---|
| Stearin | 4 Gew.-% |
| Lithiumstearat | 36 Gew.-% |
| Kaolin | 55 Gew.-% |
| Pigment green 7 | 5 Gew.-% |

Lithiumstearat sorgt für eine weiche, kreidige Abgabe. Durch den geringen Wachsgehalt ist der Abstrich gut verwischbar.

### Beispiel 7: Nachleuchtende füllstofffreie wasserfeste Farbmine im Trockenverfahren (Rohstoffe bereits verseift)

| | |
|---|---|
| Stearin | 10 Gew.-% |
| Natriumstearat | 60 Gew.-% |
| Sorbitol | 10 Gew.-% |
| Pigment (Strontiumaluminat) | 20 Gew.-% |

Die füllstofffreie Rezeptur ist semitransparent. Dadurch ist es möglich im Abstrich einen Nachleuchteffekt zu erreichen. Der hohe Bindemittelanteil ermöglicht trotz des abrasiven Pigments werkzeugschonendes Mischen, Extrudieren und Spitzen.

## Patentansprüche

1. Biologisch abbaubare und/oder kompostierbare Mine oder Kreide für Schreib-, Mal- und/oder Kosmetikzwecke enthaltend wenigstens einen Fettsäureester, wenigstens eine Metallseife und/oder Seife und wenigstens ein Farbmittel,
**dadurch gekennzeichnet, dass**
der wenigstens eine Fettsäureester und die wenigstens eine Metallseife und/oder Seife mit einem Gesamtanteil von 33 bis 90 Gew.-% enthalten sind.

2. Biologisch abbaubare und/oder kompostierbare Mine oder Kreide nach Anspruch 1,
**dadurch gekennzeichnet,**
**dass** wenigstens ein Fettsäureester und/oder wenigstens eine Metallseife und/oder Seife basierend auf natürlichen Fetten, Wachsen und/oder Ölen enthalten sind.

3. Biologisch abbaubare und/oder kompostierbare Mine oder Kreide nach Anspruch 2,
**dadurch gekennzeichnet,**
**dass** wenigstens ein Fettsäureester und/oder wenigstens eine Metallseife oder Seife basierend auf einer Carbonsäure, insbesondere einer Fettsäure wie Stearinsäure, Palmitinsäure, Ölsäure, Laurinsäure, Myristinsäure, Caprinsäure, Palmitoleinsäure, Linolsäure, Reiskleiewachs, Sonnenblumenwachs enthalten ist.

4. Biologisch abbaubare und/oder kompostierbare Mine oder Kreide nach einem der vorhergehenden Ansprüche,
**dadurch gekennzeichnet,**
**dass** wenigstens eine Metallseife und/oder Seife basierend auf Metallionen einwertiger Salze der Alkalimetalle, insbesondere Lithium, Natrium und/oder Kalium und/oder basierend auf Metallionen zweiwertiger Erdalkalimetalle, insbesondere Magnesium, Kalzium und Barium und/oder basierend auf Metallionen von Metallen, insbesondere Aluminium und Zink, enthalten ist.

5. Biologisch abbaubare und/oder kompostierbare Mine oder Kreide nach einem der vorhergehenden Ansprüche,
**dadurch gekennzeichnet,**
**dass** eine Seife basierend auf einer Fettsäure, insbesondere einer Fettsäure bis maximal C16 und/oder einer Mischung aus längerkettigen Fettsäuren als C18 und Fettsäuren bis maximal C16, und basierend auf Natrium und/oder Kalium als Metallion enthalten ist.

6. Biologisch abbaubare und/oder kompostierbare Mine oder Kreide nach einem der vorhergehenden Ansprüche,
**dadurch gekennzeichnet,**
**dass** eine Metallseife basierend auf Kationen Lithium, Magnesium, Kalzium, Barium, Aluminium und Zink als Metallion enthalten ist und/oder basierend auf Natrium und/oder Kalium als Metallion in Kombination mit einer längerkettigen Fettsäure als C18 enthalten ist.

7. Biologisch abbaubare und/oder kompostierbare Mine oder Kreide nach einem der vorhergehenden Ansprüche,
**dadurch gekennzeichnet, dass**
ein Masseverhältnis der oder des Fettsäureester(s) zu Metallseife(n) und/oder Seife(n) zwischen 1:1 bis 1:10 liegt.

8. Biologisch abbaubare und/oder kompostierbare Mine oder Kreide nach einem der vorhergehenden Ansprüche,
**dadurch gekennzeichnet, dass**
das Farbmittel mit einem Anteil von 0,1 bis 35 Gew.-%, insbesondere mit einem Anteil von 0,1 bis 25 Gew.-% enthalten ist.

9. Biologisch abbaubare und/oder kompostierbare Mine oder Kreide nach einem der vorhergehenden Ansprüche,
**dadurch gekennzeichnet, dass**
als Farbmittel Farbstoffe und/oder organische Pigmente und/oder anorganische Pigmente enthalten sind, insbesondere organische und/oder anorganische Pigmente mit einer Korngrößenverteilung D90 < 100 µm.

10. Biologisch abbaubare und/oder kompostierbare Mine oder Kreide nach einem der vorhergehenden Ansprüche,
**dadurch gekennzeichnet, dass**
wenigstens ein Füllstoff enthalten ist, insbesondere mit einem maximalen Anteil von 75 Gew.-% und/oder wobei der wenigstens eine Füllstoff insbesondere ausgewählt ist aus der Gruppe Calciumcarbonat, Kaolin, Talkum, Bimsmehl, Quarzmehl, Mica, Weißpigmente und/oder Pflanzenfasern.

11. Biologisch abbaubare und/oder kompostierbare Mine oder Kreide nach einem der vorhergehenden Ansprüche,
**dadurch gekennzeichnet, dass**
wenigstens ein Additiv enthalten ist, insbesondere mit einem maximalen Anteil von 15 Gew.-%, und/oder wobei das Additiv insbesondere ausgewählt ist aus der Gruppe Hydroxyle und/oder Carbonsäuren und/oder Salze.

12. Stift mit einer biologisch abbaubaren und/oder kompostierbaren Mine nach einem der vorhergehenden Ansprüche, insbesondere mit einer Ummantelung aus Holz oder aus einem Holzersatzwerkstoff.

13. Verfahren zur Herstellung einer biologisch abbaubaren und/oder kompostierbaren Mine oder einer Kreide nach einem der Ansprüche 1 bis 11, **dadurch gekennzeichnet, dass** der wenigstens eine Fettsäureester, die wenigstens eine Metallseife und/oder Seife oder zumindest die zu einer Metallseife und/oder Seife reagierenden Metallhydroxide und/oder Metallsalze, und das wenigstens eine Farbmittel vermischt und/oder homogenisiert, bei erhöhter, ein Schmelzen bewirkenden Temperatur thermoplastisch verarbeitet werden, und die daraus erhaltene Minengrundmasse zu Minen oder Kreidesträngen extrudiert wird, wobei der wenigstens eine Fettsäureester, die wenigstens eine Metallseife und/oder Seife oder die zur Metallseife und/oder Seife reagierenden Fettsäureester und Metallhydroxide und/oder Metallsalze derart zugegeben werden, dass die wenigstens eine Fettsäure und die wenigstens eine Metallseife und/oder Seife mit einem Anteil von 33 bis 90 Gew.-% in der Mine oder Kreide enthalten sind.

14. Verfahren zur Herstellung einer biologisch abbaubaren und/oder kompostierbaren Mine oder einer Kreide nach Anspruch 13, **dadurch gekennzeichnet, dass** Wasser zugegeben wird, welches gemeinsam mit der wenigstens einen Fettsäure, der wenigstens einen Metallseife und/oder Seife oder der zur Metallseife und/oder Seife reagierenden Fettsäuren und Metallhydroxide und/oder Metallsalze, und dem wenigstens einen Farbmittel thermoplastisch verarbeitet wird und wobei der Anteil des Wassers während der thermoplastischen Verarbeitung auf höchstens 0,5 Gew.-%, vorzugsweise auf 0 Gew.-% reduziert wird.

15. Verfahren zur Herstellung einer biologisch abbaubaren und/oder kompostierbaren Mine oder einer Kreide nach einem der Ansprüche 13 oder 14, **dadurch gekennzeichnet**,
die wenigstens eine Fettsäure, die wenigstens eine Metallseife und/oder Seife oder die zur Metallseife und/oder Seife reagierenden Metallhydroxide, und das wenigstens eine Farbmittel bei einer Temperatur von mindestens 100°C - 150°C vermischt werden und bei 50°C bis 140°C extrudiert werden.
